# EUROPEAN PATENT APPLICATION

(11) **EP 1 671 630 A2**
(43) Date of publication of application: **21.06.2006**
(21) Application number: 06007096.8
(22) Date of filing: 04.02.2000
(51) Int. Cl.: A61K 31/195, A61K 31/495, A61K 31/505, A61K 31/198

(54) **L-Arginine based formulations for treating diseases and methods of using the same**

(30) Priority: 05.02.1999 US 118903 P
(62) Divisional of application: 00911701.1
(71) Applicant: Angiogenix, Inc., Burlingame, CA 94010 (US)
(72) Inventor: The designation of the inventor has not yet been filed
(74) Representative: Coret, Sophie V.G.A.

(57) **Abstract**

A therapeutic mixture comprised of L-arginine and a NOS agonist (e.g. Doxazosin) is disclosed for the treatment of diseases.

## Description

### FIELD OF THE INVENTION

The present invention relates generally to the impact of arginine supplementation on various diseased states. In certain diseased states, unpredictably L-arginine supplementation alone may positively impact the diseased states (i.e., hyperhomocysteinemia). More generally, the present invention is directed to a combination of arginine and other agents which have been found to deplete the bioavailability of arginine.

### BACKGROUND OF THE INVENTION

One approach to treating cardiac disease is to effect the dilation of vascular conduits in the body. In this regard, nitric oxide has been shown to be formed enzymatically as a normal metabolite from arginine in the vascular endothelium and provides an important component to the formation of Endothelium-Derived Relaxing Factor (EDRF). EDRF appears to be equivalent to Endothelium Derived Nitric Oxide (EDNO) and as used herein EDRF and EDNO are used interchangeably unless otherwise indicated.

It has also been established that a family of enzymes called Nitric Oxide Synthase ("NOS") form nitric oxide from L-arginine, and the nitric oxide produced is responsible for the endothelium dependent relaxation and activation of soluble guanylate cyclase, neurotransmission in the central and peripheral nervous systems, and activated macrophage cytotoxicity. Nitric Oxide Synthase occurs in many distinct isoforms which include a constitutive form ("cNOS") and an inducible form (iNOS). The constitutive form is present in normal endothelial cells, brain, neurons and some other tissues. Formation of nitric oxide by the constitutive form in endothelial cells is thought to play an important role in normal blood pressure regulation, prevention of endothelial dysfunction such as hyperlipodemia, arteriosclerosis, thrombosis, and restenosis. A by-product of the conversion of L-arginine is L-citrulline. Brain, endothelium, and macrophage isoforms of NOS appear to be products of a variety of genes that have approximately 50% amino acid identity. NOS in brain and in endothelium have very similar properties, the major differences being that brain NOS is cytosolic and the endothelial enzyme is mainly a membrane-associated protein.

Functionally, the constitutive form of Nitric Oxide Synthase, which is the predominant synthase present in brain and endothelium, may be active under basal conditions and can be further stimulated by increases in intracellular calcium that occur in response to receptor-mediated agonists or calcium ionophores. cNOS appears to be the "physiological" form of the enzyme and plays a role in a diverse group of biologic processes. In vitro studies suggest that the activity of NOS can be regulated in a negative feedback manner by nitric oxide itself. In cardiocerebrorenovascular circulation, the primary target for constitutively produced nitric oxide is believed to be soluble guanylate cyclase located in vascular smooth muscle, the myocardium (myocytes) and coronary vascular smooth muscle.

In contrast to the cNOS, the inducible, calcium-independent form, iNOS was initially only described in macrophages. It is now known that induction of Nitric Oxide Synthase can occur in response to appropriate stimuli in many other cell types. This includes both cells that normally do not express a constitutive form of nitric oxide synthase, such as vascular smooth muscle cells, as well as cells such as those of the myocardium that express considerable levels of the constitutive isoform. The inducible form of nitric oxide synthase has been found to be induced in vascular smooth muscle cells, for example, by various cytokines and/or microbial products.

iNOS exhibits negligible activity under basal conditions, but in response to factors such as lipopolysaccharide and certain cytokines, expression occurs over a period of hours. The induced form of the enzyme produces much greater amounts of NO than the constitutive form, and induced NOS appears to be the "pathophysiological" form of the enzyme because high concentrations of NO produced by iNOS can be toxic to cells. Induction of iNOS can be inhibited-by glucocorticoids and some cytokines.

Activity of endothelial cell NOS (eNOS) has a well-established role in maintaining normal vascular tone. eNOS and its substrate, L-arginine, may have been shown to have important vasoprotective mechanisms in addition to their role in maintaining normal blood pressure. Most significantly, treatment with L-arginine or with other agents that increase eNOS activity and NO production have been found to protect against ischemia-reperfusion injury in various experimental models. Since these treatments also stimulate tPA (tissue plasminogen activator) production and/or inhibit production of plasminogen activator inhibitor-1, it is likely that their protective effects are due at least in part to effects in increasing tPA activity. Recently, many agents whose principal actions are unrelated to eNOS activity have been shown to have independent auxiliary actions through eNOS activation and NO production. These include organic nitrates, converting enzyme inhibitors, amrinone, nevilolol, S-nitroso-tPA, pravastatin and amlodipine. Doxazosin appears to have a similar auxiliary mechanism in that it increases tPA levels as a result of effects on eNOS activation in endothelial cells ("ECs").

The term "subject" is used herein to mean any mammal, including humans, where nitric oxide formation from arginine occurs. The methods herein for use on subjects contemplate prophylactic use as well as curative use.

The term "endpoints" as used herein refers to clinical events encountered in the course of treating cardiovascular disease, up to and including death (mortality).

As used herein, the term "pharmaceutically acceptable carrier" refers to a carrier medium which does not interfere with the effectiveness of the biological activity of the active ingredients and which is not toxic to the hosts to which it is administered.

As used herein, the term "about" means plus or minus 10% of the number to which reference is being made.

As used herein, the term "prophylactic or therapeutic" treatment refers to administration to the host of the biologically active agent(s) either before or after onset of the biological damage to the host. If the biological agent(s) are administered prior to exposure to the agent causing the biological damage, the treatment is prophylactic (i.e., it protects the host against the damage), whereas if it is administered after exposure to the agent causing the damage, the treatment is therapeutic (i.e., it alleviates the existing damage).

L-arginine as used herein includes all biochemical equivalents (i.e. salts, precursors, and its basic form). "To mix", "mixing", or "mixture(s)" as used herein means mixing a substrate (i.e. L-arginine) and an agonist (*e.g.*, Doxazosin or DOX): 1) prior to administration ("in vitro mixing"); 2) mixing by simultaneous and/or consecutive, but separate (i.e. separate intravenous lines) administration of substrate (*i.e.*, L-arginine) and agonist of NOS or an agent which depletes natural levels of L-arginine (*e.g*., DOX) to cause "in vivo mixing"); and 3) the administration of a NOS agonist after saturation with a NOS substrate (i.e. L-arginine is administered to build up a supply in the body prior to administering the NOS agonist (again, *e.g*., nitroglycerin or DOX)); or any combination of the above which results in the delivery of therapeutic amounts of a NOS agonist and a NOS substrate in an additive or synergistic way with regard to the treatment of disease, preferably cardiocerebrorenovascular disease.

Agonist refers to an agent which increases NO production by increasing the bio-transformation of L-arginine either through enzymatic activation or increasing gene expression (*e.g*., by increasing total tissue content of NOS). Of course, either or both of these mechanisms may be acting simultaneously. Although this may not be direct stimulation of NOS, for purposes herein, the term "agonist" is meant to include any agent which depletes the bioavailability of L-arginine in a mammal. NOS agonists may include those described in U.S. Patent No. 5,543,430, U.S. Patent No. 5,767,160, U.S. Patent Appln. No. 08/833,842 and Kaesemeyer et al., JACC, Vol. 33, No. 1, ppgs. 234-41, each of which are hereby incorporated by reference in their entirety.

The present invention is preferably useful in preventing, treating, arresting, or ameliorating disease conditions which are benefited by the bio-transformation of a substrate into nitric oxide or "native" nitric oxide.

The present invention may also be useful in preventing, treating, arresting, or ameliorating disease conditions which are benefited by the bio-transformation of L-arginine into "native" nitric oxide through enzyme activation of NOS.

The present invention may also be useful in achieving a beneficial effect when treating disease conditions by increasing or maximizing the production of EDRF or EDNO, and reducing clinical endpoints to include mortality.

The present invention may also be useful in preventing, treating, or avoiding tachycardia and ischemia.

The present invention may also be useful in preventing, treating, or avoiding diseases associated with free radical production, specifically superoxide anion (O₂^{•-}).

### SUMMARY OF THE INVENTION

The present invention may also be useful in preventing, treating, arresting, or ameliorating reperfusion injury in subjects who have had abrupt restoration of blood flow.

One embodiment of the present invention is directed to the administration of a biological equivalent of arginine and an agonist of NOS (*e.g*., DOX) to produce a beneficial effect.

The mixture of L-arginine and DOX may be particularly useful for the treatment of hypertension, hypertensive heart disease, coronary heart disease, including arteriosclerosis, angina, myocardial infarction, coronary thrombosis, restenosis post angioplasty, and sudden death, as well as a wide range of cardiovascular disease (heart failure, stroke, hypercholesterolemia, and peripheral vascular diseases), and renovascular ischemia/hypertension.

In an alternative embodiment of the invention, therapeutically effective amounts of a precursor of EDNO and an agonist of NOS (*e.g*., DOX) are combined prior to administration to a patient. In a more preferred embodiment of the present invention, therapeutically effective amounts of L-arginine and therapeutically effective amounts of DOX are mixed at a physiologically acceptable pH.

Another embodiment of the present invention is a method for treating hypertension in a subject by vasodilation or vasorelaxation comprising: selecting a hypertensive subject; administering L-arginine and a NOS agonist (*e.g*., DOX) to the subject; obtaining periodic blood pressure measurements of the subject; and continuing administration of the formulation until a desirable blood pressure or therapeutic effect is detected in the subject. A desirable blood pressure in a hypertensive subject should ultimately be within the following ranges: systolic preferably in the range of 95-180 mmHg, more preferably in the range of 105-165 mmHg, and even more preferably in the range of 120 to 140 mmHg; and diastolic preferably in the range of 55-115 mmHg, more preferably in the range of 65-100 mmHg, and even more preferably in the range of 70 to 90 mmHg, and most preferably 75-85 mmHg. Under no circumstances should the systolic be permitted to go below 95 mmHg.

Another embodiment of the present invention is a method for preventing or treating cardiovascular disease in a non-hypertensive subject by vasodilation or vasorelaxation comprising: selecting a subject; administering to said subject a formulation comprising a mixture of DOX and an endothelium dependent source or precursor of nitric oxide (*e.g*., L-arginine); obtaining periodic measurements of vasorelaxation on the subject and; continuing administration of the formulation until a desirable state of vasorelaxation or desirable therapeutic effect is detected in the subject.

Another embodiment of the present invention is a method for stimulating cNOS in a subject which comprises: selecting a subject; administering to said subject a formulation comprising a mixture of L-arginine and DOX so as to increase "native" NO production and reduce endpoints to include mortality.

In summary, the present invention resides broadly in a therapeutic mixture of Doxazosin and a substrate of NOS. The substrate of NOS can be a biological equivalent of L-arginine or L-arginine.

Another feature of the invention resides broadly in a method of treating a disease condition in a subject by vasodilation or vasorelaxation comprising: selecting a subject; administering a mixture of L-arginine and Doxazosin; obtaining periodic indicators of vasorelaxations for the subject; and continuing administration of the mixture until a desirable state of vasorelaxation is obtained. The mixture can be administered intravenously, buccal, intracoronary, intraarterially, intramuscularly, topically, intranasally, rectally, sublingually, orally, subcutaneously, by patch or inhalation. The disease can be hypertension, hypercholesterolemia, hypertensive heart disease, coronary heart disease, cardiovascular disease, cerebrovascular disease, and renovascular disease. The coronary heart disease can be restenosis post angioplasty. The L-arginine and Doxazosin can be mixed in vivo. The L-arginine and Doxazosin can be administered at a therapeutic concentration. The therapeutic concentration of L-arginine can be from 7.5% to about 30% w/v (g/ml).

A further feature of the present invention is a method of stimulating nitric oxide synthase to produce nitric oxide, said method comprising: administering L-arginine and an agonist of nitric oxide synthase to a subject have a nitric oxide synthase receptor site, said agonist being Doxazosin; and stimulating said nitric oxide synthase to a desirable level with said agonist of nitric oxide synthase. The L-arginine is preferably in excess of Doxazosin. The therapeutically effective amount of L-arginine is preferably combined with a therapeutically effective amount of Doxazosin prior to administration to the patient.

A further feature of the invention resides in a formulation comprised of a non-pharmaceutical or nutraceutical agonist of NOS and a biological equivalent of L-arginine, said agonist being selected from the group consisting of hawthorne extract, gingko biloba, allicin (garlic), melatonin, folic acid, elderberry extract, grape pip riboflavin, phytoestrogens, soy isoflavones, resveraterol and quercetin. The mixture is preferably comprised of L-arginine and a NOS agonist, the NOS agonist being a selective alpha-1 blocker.

A further feature of the invention resides broadly in a method of treating a diseased state comprised of administering a mixture of L-arginine and an agonist of NOS; preferably further comprising the administration of a selective alpha-1 blocker. The selective alpha-1 blocker is preferably prazosin or terazosin.

### BRIEF DESCRIPTION OF THE DRAWINGS

Fig. 1A is a top portion of a schematic representation of select compounds and the conversion of L-arginine into NO and the proposed L-arginine dependent and independent pathways.
Fig. 1B is a schematic representation of the proposed L-arginine dependent and independent pathways.
Fig. 2 is a schematic representation of the dynamics of LA supply to NOS.
Fig. 3 indicates the effect of B^{α+} and y⁺ transporters on cellular uptake of [³H]-L-arginine.
Fig. 4 indicates the effect of bradykinin (BK, 1αM) on y⁺ transport of [³H]-LA in bovine aortic endothelial cells.
Fig. 5 indicates the effect of substance P (SP, 1 µM) on y⁺ transport of [³H]-LA in bovine aortic endothelial cells.
Fig. 6 indicates the effect of acetylcholine (Ach, 5 µM) on y⁺ transport of [³H]-LA in bovine aortic endothelial cells.
Fig. 7 indicates the effect of s-nitroso-acetyl-penicillamin (SNAP, 200 µM; equivalent to 0.4 µM NO) on y⁺ transport of [³H]-LA in bovine aortic endothelial cells.
Fig. 8 indicates the effect of dipropylenetriamine NONOate (DPTA, 10-0.01 µM; equivalent to 20-0.02 µM NO) on y⁺ transport of [³H]-LA in bovine aortic endothelial cells.
Fig. 9 indicates the effect of L-arginine (LA, 5 x 10⁻⁴M) and n-ω-nitro-L-arginine methyl ester (L-NAME, 5 x 10⁻⁴M) on substance P (SP, 1 µM) or calcium ionaphore, A-23187 (CI, 1 µM) induced superoxide anion (O₂^{•-}) formation in bovine aortic endothelial cells (BAEC).

### DETAILED DESCRIPTION OF THE PREFERRED EMBODIMENTS

Research into the area of NOS activation reveals a number of agonists of NOS. These include nitroglycerin, the statins (pravastatin, lovastatin, fluvastatin), aspirin, pycrogenol, L-sepiaterin•H₂O, n-acetyl cysteine, reduced glutathione, amiodarone, nifedipine, felodipine, and Doxazosin, and certain nutraceuticals (e.g., hawthorne extract, gingko biloba, allicin (garlic), melatonin, folic acid, elderberry extract, grape pip sensing riboflavin, phytoestrogens, soy isoflavones, resveraterol and quercetin (red wine phenolics or polyphenols)).

Doxazosin, a selective alpha-1 blocker, is pharmacologically similar to prazosin and terazosin. It has been shown that selective alpha-1 blockers lower total and LDL cholesterol. Thus, these selective alpha-1 blockers may be useful in treating both hypertension and hypercholesterolemia. Doxazosin (DOX), an effective antihypertensive agent and α - adrenoreceptor antagonist, has been found to increase serum levels of tissue plasminogen activator (tPA). In fact, a wide variety of vasoactive agents (e.g. bradykinin, muscarinic agonists and growth factors) which increase tPA levels are also agonists of nitric oxide synthase (NOS). DOX activity as a NOS agonist was investigated in cultured bovine aortic EC using two methods to assess NO production: conversion of oxyhemoglobin to methamoglobin and a NO sensitive electrode. It was found that DOX (10⁻⁷ - 10⁻⁵ M) produced a dose-related increase (64-145%) in NO production. This increase in NO to DOX (10⁻⁶ M) was blocked by 72% by prior administration of the NOS inhibitor, L-NAME (5 x 10⁻⁴ M). In addition, the NO responses were accentuated by the presence of supplemental L-arginine (5 x 10⁻⁴ M) by 65%. Acetylcholine also produced a dose-related increase in NO production. This increase in NO to ACH (10⁻⁶ M) was blocked by 86% by prior administration of L-NAME. In addition, the NO responses were accentuated by 72% in the presence of supplemental L-arginine. DOX, therefore, appears to be a NOS agonist in EC.

### Doxazosin methods and general protocol

For all experiments, human coronary artery endothelial cells (passage 3-5 - Clonetics) were maintained at 37°, 95% O₂ and 5% CO₂ in Medium 199 (M199) supplemented with 5% fetal bovine serum (FBS, Hyclone), 10% iron supplemented FBS (Hyclone), thymidine (100mg mL⁻¹), penicillin G (100U mL⁻¹) and streptomycin (100µg mL⁻¹).

In all experiments, the effects of pretreatment with L-NAME (10⁻³ M) in blocking the actions of DOX, and excess L-arginine (10⁻³ M) in reversing any L-NAME effect was examined. Acetylcholine, an eNOS agonist, was used in all experiments as the positive control at concentrations of 10⁻⁷ and 10⁻⁶ M.

### Nitric Oxide Measurements

Methemoglobin - The effect of DOX on NO production in EC was determined using a photometric assay for conversion of oxyhemoglobin to methemoglobin. For this assay, EC grown to confluency on microcarrier beads are placed into a water-jacketed chromatography column and superfused with a Kreb's-Ringer buffer containing 3 µM oxyhemoglobin and 50 µM LA (L-arginine). Perfusate is then directed into a flow-through cuvette in a dual wavelength spectrophotometer and change in absorbancy (415/405 nm) is measured. Experimental stimulation was carried out by 3 min infusion periods of DOX added to buffer perfusion to yield final concentrations of 10⁻⁷ and 10⁻⁶ M. For analysis, we determined the area under the curve for the change in absorbancy response/min caused by DOX assuming a one to one correspondence for NO and metHb production, the known stoichiometric balance for this reaction. No electrode - NO production was measured with a NO meter connected to a polargraphic NO electrode as previously described. The NO sensor probe will be inserted vertically into 24-well plates containing confluent EC such that the tip of the electrode is submerged 2 mm under the surface of medium (above - 1ml). The reaction was initiated when desired concentrations of DOX are added to the well. Calibrations were performed with S-nitroso-acetyl-penicillamine.

### tPA assay

In the tPA assay, EC was grown to confluency in 24-well plates and on experimental days, the medium was discarded and replaced with 0.5ml serum-free M199 containing 1% BSA and 50µM LA and incubated at 37% for 48 hrs in the presence of DOX (10⁻⁷ to 10⁻⁵ M) or acetylcholine (10⁻⁷ and 10⁻⁶ M) with and without L-NAME and excess LA. After incubation, the medium was harvested for determination of tPA content by an ELISA kit.

It would appear that DOX, like nitroglycerin, substance P and bradykinin, acts as a NOS agonist. It appears that the responses to DOX can be magnified significantly with L-arginine supplementation. It appears the overall therapeutic result with DOX is augmented to the extent they act as agonists of NOS. The fact that DOX is an agonist or a stimulator of nitric oxide synthase has important implications. Mixing DOX "in vitro" or "in vivo" with L-arginine may have an unforeseen beneficial effect that is associated with excess L-arginine providing additional substrate for NOS and the NOS being catalyzed to enzymatically increase the bio-transformation of L-arginine into nitric oxide (EDRF or EDNO) which would in turn amplify the overall therapeutic effect.

Stimulation of NOS by DOX in the presence of excess L-arginine or other substrate precursor of native NO may be used to prevent, treat, arrest, or ameliorate any disease or condition which is positively affected by NO production. Such conditions include hypertensive cardiocerebrorenovascular diseases and their symptoms as well as non-hypertensive cardiocerebrorenovascular diseases. The mixture is particularly useful for subjects in need of native NO production for therapeutic angiogenesis. Application of such a mixture is beneficial for: (1) chronic stable angina; (2) unstable angina; (3) acute myocardial infarction; (4) hibernating myocardium; (5) stunned myocardium; (6) limitation of ventricular remodeling in post myocardial infarction and subsequent risk of congestive heart failure; (7) prophylaxis of recurrent myocardial infarction; (8) prevention of sudden death following myocardial infarction; (9) vasospastic angina; (10) congestive heart failure-systolic seen in association with 1-6 above; (11) congestive heart failure-diastolic seen in association with 1-10 above and 12-15 below; (12) microvascular angina seen in association with 1-11 above and 15 and 16 below; (13) silent ischemia seen in association with 1-12 above and 15 and 16 below; (14) reduction of ventricular ectopic activity seen in association with 1-13 above and 15 below; (15) any or all of the above 1-14 states of ischemic myocardium associated with hypertensive heart disease and impaired coronary vasodilator reserve; (16) control of blood pressure in the treatment of hypertensive crisis, perioperative hypertension, uncomplicated essential hypertension and secondary hypertension; (17) regression of left ventricular hypertrophy seen in association with 15 and 16 above; (18) prevention and or regression of epicardial coronary arteriosclerosis seen in 1-17 above; (19) prevention of restenosis post angioplasty; (20) prevention and/or amelioration of free radical mediated reperfusion injury in association with 1-19 above; (21) use of the combination in the prevention of myocardial injury during cardioplegic arrest during coronary bypass or other open heart surgery i.e. use of the combination as a cardioplegic solution; (22) post transplant cardiomyopathy; (23) renovascular ischemia; (24) cerebrovascular ischemia (TIA) and stroke; (25) pulmonary hypertension; and (26) peripheral vascular disease (claudication).

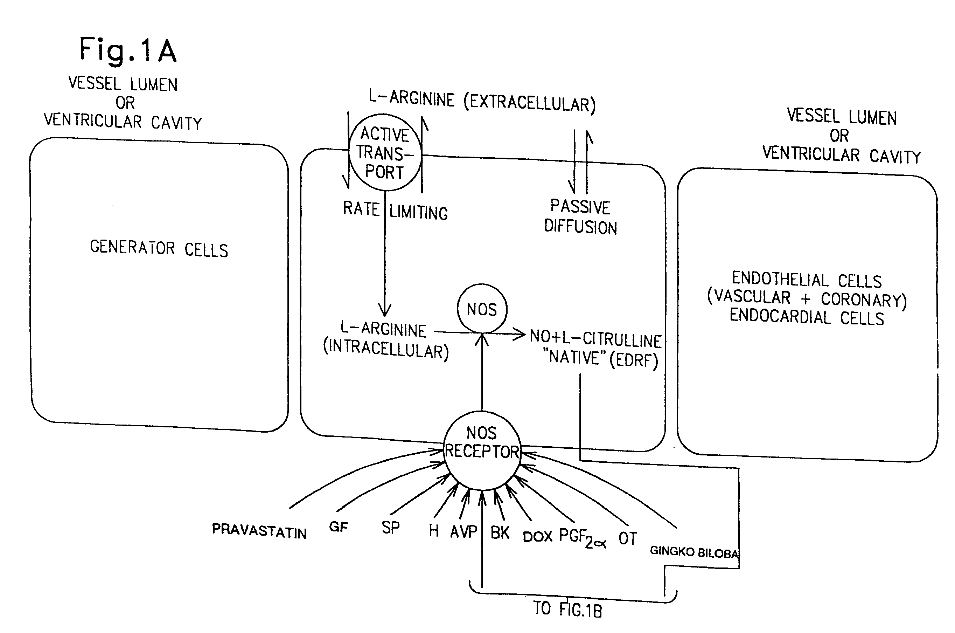

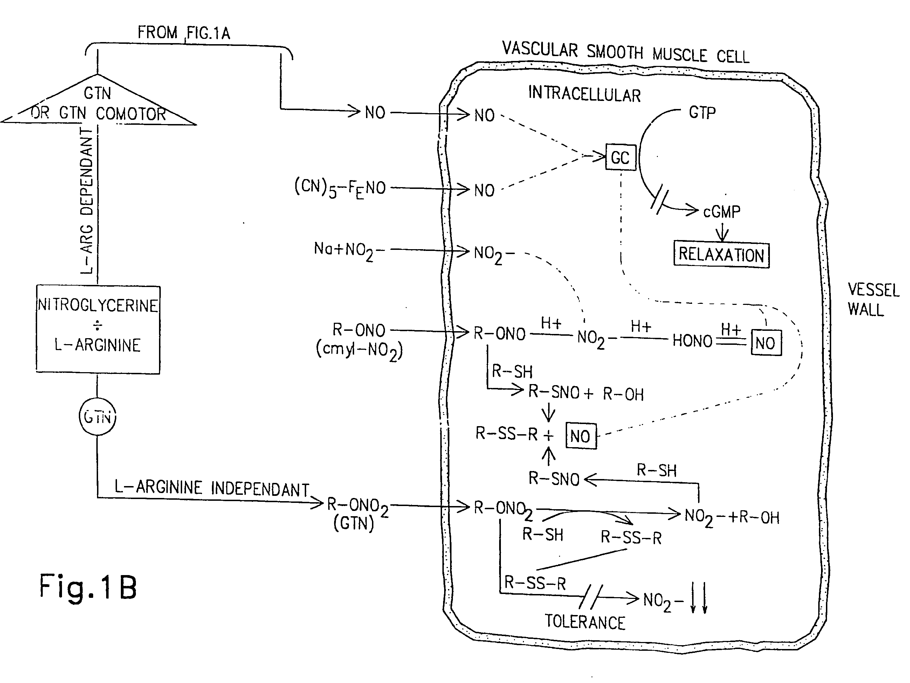

Vascular smooth muscle cells are located mainly in veins, arteries, and coronary arteries. The following discussion focuses on smooth muscle and myocyte relaxation stimulated by vasodilators but should not be so limited. The present invention is useful when NO regulation is beneficial. As discussed above the Nitric Oxide Synthase in the cells is normally cNOS, the constitutive form of Nitric Oxide Synthase, and the generator cells are endothelial cells and the target cells are vascular smooth muscle cells. Fig. 1A and Fig. 1B are schematic illustrations of a proposed mechanism of action of preferred substances (e.g., DOX) and arginine and are not intended to imply any cellular relationship or geography of the various sites of action, but rather meant to illustrate their functional relationship. Although Fig. 1 lists certain preferred agents, it is meant as a representative sampling of DOX. In Fig. 1A and Fig. 1B the abbreviation SP represents Substance P and the abbreviation GF represents select Growth Factors.

A preferred combination to be employed is a mixture that involves therapeutic concentrations of L-arginine and therapeutic concentrations of DOX. Any pharmaceutical grade L-arginine will be sufficient and should be diluted preferably to 2.5-60% w/v (g/ml), more preferably to 5-45% w/v (g/ml), even more preferably between 7.5-30% w/v (g/ml), even more preferably to 10-15% w/v (g/ml), and most preferably 10% w/v (g/ml) L-arginine. The typical doses anticipated will be 30 grams of L-arginine in sterile water (Total Volume 300 cc). L-arginine is anticipated eventually to be approximately 10:1 to about 25:1 of the hydrochloride salt to L-arginine as a base, and even more preferably 15:1 to about 20:1 hydrochloride salt to base, and most preferably 15:1 hydrochloride salt to base. In this example 28 to 29 grams will be the hydrochloride salt and 1 to 2 grams of L-arginine will be base.

In the preferred embodiment discussed herein, L-arginine is used in conjunction with DOX. As part of a "mixture", DOX is included together with L-arginine at clinically effective weight ratios of between 1:2 to 1:150. Even more particularly, the ratio of DOX to L-arginine in the formulation is between 1:5 to 1:100. The most preferred embodiment of the "mixture" is the ratio of DOX to L-arginine at 1:50.

The ratio of DOX to L-arginine is preferably within the range 1:2 to 1:50, Wt/Wt. For example, DOX/L-arginine at a ratio of 1:2 would include 40 mg/day DOX with 80 mg/day L-arginine. Where the ratio of DOX/ L-arginine is at a ratio of 1:20, for example, 20 mg/day DOX would be administered with 400 mg/day L-arginine. The amounts above have been found to be effective, however, each route of administration (i.e. IV, oral, transdermal, intracoronary, intra-arterial, etc.) may vary in their requirements.

Even more particularly, the presently disclosed "mixtures" may be described in terms of their relative concentrations (grams) administered as part of a continuous intracoronary, intra-arterial, intravenous and intrapericardial infusions. In one particular embodiment, the formulation is administered as a mixture of DOX with L-arginine encased in liposomes so as to provide maximum retention time of the mixture in any given vascular bed being perfused by a catheter delivering the DOX/ L-arginine mixture. In some cases the liposomes containing the mixture of DOX and L-arginine may also contain genetic material which will code for the synthesis of the growth factor following transfection of the genetic material into the surrounding tissue of the vascular bed. In some cases pellets containing the aforementioned mixtures may be directly implanted into the myocardium at the time of coronary bypass graft surgery. In yet another case, a therapeutic mixture of L-arginine and DOX may be repeatedly infused into the pericardial space via an indwelling infusion catheter.

While not wishing to be bound by theory, it is now believed that DOX has a stimulating effect on cNOS, and furthermore, that this action to stimulate cNOS is involved in the angiogenic response seen with DOX. Thus, DOX and L-arginine appear to have a heretofore unexpected additive and/or synergistic effect on cNOS stimulation. The stimulation of cNOS may be a result of cNOS having a unique receptor site for DOX or DOX may initiate a cascade of events which stimulate NOS. Administering the two also provides adequate substrate for cNOS processing of L-arginine since the L-arginine is added in excess while at the same time stimulating the enzymatic activity of NOS. Whether it is a synergistic effect or additive effect, what is clear is that "mixing" a precursor substrate of "native" nitric oxide with DOX results in a heretofore unexpected increase in NO production.

By way of example, a therapeutic mixture, as previously described, of L-arginine and DOX may be infused into the coronary arteries of a patient with chest pain at the time of a coronary arteriogram which reveals multiple diffuse arthrosclerotic obstructive lesions which are amenable to treatment with bypass surgery or angioplasty. The treatment is expected to result in improvement in collateral blood flow and a decrease in the severity and frequency of angina attacks over the next 6 to 12 months.

The methods of the present invention involve administering to a mammalian host, preferably a human host, pharmacologically effective amounts of arginine and a NOS agonist such as DOX. The agents may be combined in vitro before administration or separately administered, either concurrently or simultaneously, with administration generally taking place up to 24 hours before or after the administration of the other biological active agent(s).

The administration(s) may take place by any suitable technique, including oral, subcutaneous and parenteral administration, preferably parenteral or oral. Examples of parenteral administration include intravenous, intra-arterial, intramuscular, and intraperitoneal. The dose and dosage regimen will depend mainly on whether the inhibitors are being administered for therapeutic or prophylactic purposes, separately or as a mixture, the type of biological damage and host, the history of the host, and the type of inhibitors or biologically active agent. The amount must be effective to achieve an enhanced therapeutic index. It is noted that humans are generally treated longer than the mice and rats with a length proportional to the length of the disease process and drug effectiveness. The doses may be single doses or multiple doses over a period of several days. Therapeutic purpose is achieved as defined herein when the treated hosts exhibit improvement against disease or infection, including but not limited to improved survival rate, more rapid recovery, or improvement or elimination of symptoms. If multiple doses are employed, as preferred, the frequency of administration will depend, for example, on the type of host and type of disease, dosage amounts, etc. The practitioner may need to ascertain upon routine experimentation which route of administration and frequency of administration are most effective in any particular case.

Compounds and agents (*e.g.*, both L-arginine and DOX) of the present invention, in conjunction with a pharmaceutically acceptable carrier, may be used for any of the therapeutic effects, discussed above. Such compositions may be in the form of an agent(s) in combination with at least one other agent, such as stabilizing compound, which may be administered in any sterile, bio-compatible pharmaceutical carrier, including, but not limited to, saline, buffered saline, dextrose, and water. The compositions may be administered to a patient alone, or in combination with other agents, drugs or hormones. Pharmaceutically-acceptable carriers may also be comprised of excipients and auxiliaries which facilitate processing of the active compounds into preparations which can be used pharmaceutically. Further details on techniques for formulation and administration may be found in the latest edition of Remington's Pharmaceutical Sciences (Maack Publishing Co., Easton, PA) hereby incorporated herein by reference in its entirety.

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combination of active compounds with solid excipient, optionally grinding a resulting mixture, and processing the mixture of granules, after adding suitable auxiliaries, if desired, to obtain tablets or dragee cores. Suitable excipients are carbohydrate or protein fillers, such as sugars, including lactose, sucrose, mannitol, or sorbitol; starch from corn, wheat, rice, potato, or other plants; cellulose, such as methyl cellulose, hydroxypropylmethyl-cellulose, or sodium carboxymethylcellulose; gums including arabic and tragacanth; and proteins such as gelatin and collagen. If desired, disintegrating or solubilizing agents may be added, such as the cross-linked polyvinyl pyrrolidone, agar, alginic acid, or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound. i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with a filler or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks's solution, Ringer's solution, or physiologically buffered saline. Aqueous injection suspensions may contain substances which increase the viscosity of the suspension, such as sodium carboxyrnethyl cellulose, sorbitol, or dextran. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Optionally, the suspension may also contain suitable stabilizers or agents which increase the solubility of the compounds to allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, succinic, etc. Salts tend to be more soluble in aqueous or other protonic solvents than are the corresponding free base forms. In other cases, the preferred preparation may be a lyophilized powder which may contain any or all of the following: 1-50 mM histidine, 0.1%-2% sucrose, and 2-7% mannitol, at a pH range of 4.5 to 5.5, that is combined with buffer prior to use.

After pharmaceutical compositions have been prepared, they can be placed in an appropriate container and labeled for treatment of an indicated condition. Such labeling would include amount, frequency, and method of administration.

The therapeutically effective dose of DOX can be estimated initially either in cell culture assays, e.g., of neoplastic cells, or in animal models, usually mice, rabbits, dogs, or pigs. The animal model may also be used to determine the appropriate concentration range and route of administration. Such information can then be used to determine useful doses and routes for administration in humans.

The exact dosage will be determined by the practitioner, in light of factors related to the subject that requires treatment. Dosage and administration are adjusted to provide sufficient levels of the active moiety or to maintain the desired effect. Factors which may be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions may be administered every 3 to 4 days, every week, or once every two weeks depending on half-life and clearance rate of the particular formulation.

A second aspect of the present invention is based on a beneficial affect of L-arginine. As discussed above, intracellular L-arginine (LA) is the substrate required by nitric oxide synthase (NOS) to liberate nitric oxide (NO). However, when cellular supply of LA is limited, NOS utilizes molecular oxygen as a lone substrate producing superoxide anion (O₂^{•-}) and other reactive free radicals which can lead to cardiovascular dysfunction and the pathogenesis of disease.

The total intracellular concentration of LA (0.1 - 1mM) in endothelial cells (EC) greatly exceeds the Kₘ of eNOS for LA (-3 µM). This suggests that eNOS is saturated with substrate and that levels of intracellular LA are not limiting for NO production. However, other studies have shown that availability of LA varies greatly within the EC due to intracellular compartmentalization and dequestration in addition to degradation by arginase or the presence of endogenous inhibitors of eNOS (i.e., asymmetrical dimethylarginine). Recently, it has also been shown that concurrent cellular LA transport may be more important than intracellular LA levels in providing LA to NOS for NO production. Therefore, total intracellular concentration of LA may not truly reflect the LA available at the site of NOS action.

Supply of LA may become limiting and reduce formation of NO in normal and pathological states. Treatment of guinea pigs with LA has been shown to increase the duration of the vasodilatory response to acetylcholine under normal physiological conditions; prior stress with norepinephrine infusion accentuates this enhancement process. It has been demonstrated that acetylcholine and a Ca⁺⁺ -ionophore which release NO can induce tolerance in isolated arterial rings. Tolerance was associated with depletion of LA and was reversed with LA repletion. LA may also become limiting under pathologic conditions. Endothelial dysfunction in cardiomyopathic hamsters can be reversed by LA . In addition, humans with acute hyperglycemia exhibit intense vasoconstriction and impaired endothelial function which can be completely reversed by intravenous infusions of low concentrations of LA. Other diseases in which pathology is attributed to a deficiency of LA include hypertension, atherosclerosis, restenosis - post coronary angioplasty and reperfusion injury. Similarly, addition of LA in these circumstances also ameliorates the deficit in endothelium-dependent relaxation.

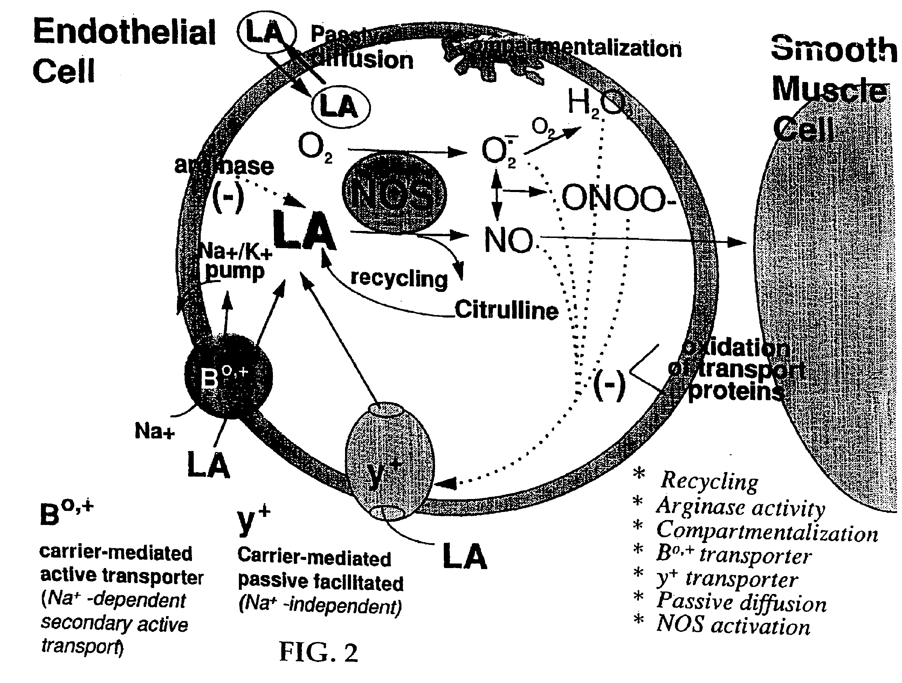

Intracellular LA is derived from several sources including the transport of LA into cells, amount of intracellular L-citrulline recycled back to LA, rate of degradation of LA (arginase), incorporation of LA into proteins (compartmentalization) and the amount of LA utilized upon activation of intracellular NOS. Uptake of LA into EC occurs through two carrier-mediated transporters and passive diffusion. The saturable carrier-mediated transporters include a sodium-dependent active transporter, system B^{α+} and a sodium-dependent transporter, system y⁺ (Fig. 2). The majority (80%) of LA delivered into most cells is through the y⁺ transporter. Regulation of LA transport appears to involve cellular membrane potential. Exposure of endothelial cells to hyperpolarizing agents including ATP and bradykinin increases LA uptake while a decrease in LA transport was observed when cells were treated with agents that cause cellular depolarization. In addition, factors that reduce the activity of the y⁺ transporter, including free radicals, may also reduce LA available for NOS.

When the balance of transporter regulatory factors is negative, LA supply becomes limiting and subsequent production of O₂^{•-} may contribute to vascular and organ pathology. We compared the effects of NOS agonists and NO donors on LA uptake by EC. Effects of NOS stimulation on superoxide anion production were also assessed in the presence and absence of LA and the NOS antagonist, L-NAME.

*Materials and Methods - Drugs and chemicals.* Primary culture BAEC from fresh blood vessels were obtained through a local slaughter house (Augusta, Georgia). Cell culture medium M-199 and penicillin/streptomycin were purchased from Gibco (Gaithersburg, MD), fetal bovine serum and iron supplemented calf serum from Hyclone (Logan, UT), and *Thermanox* coverslips from Fisher Scientific (Pittsburgh, PA). The NO donor dipropylenetriamine NONOate (DPTA) was purchased from Calbiochem (LaJolla, CA), L-[2,3,4,5-³H]-arginine monohydrochloride (specific activity 2.26 Tbq/mmol; 61.0 Ci/mmol) from Amersham (Arlington Heights, IL) and Ecoscint-A scintillation fluid from National Diagnostics (Atlanta, GA). In addition to s-nitroso-acetyl-penicillamide (SNAP), NOS agonists substance P (SP), bradykinin (BK) and acetylcholine (Ach) and all other chemical reagents were purchased from Sigma Chemical Company (St. Louis, MO).

*Characterization of L-arginine transport system in bovine aortic endothelial cells*. Bovine aortic endothelial cells (BAEC, passages 2-6) were maintained in 100 mm dishes using medium M-199 supplemented with 5% fetal bovine serum, 15% iron supplemented bovine calf serum and penicillin-streptomycin. Prior to experiments, cells were split 1:4, transferred into 24 well plates, and allowed to grow to confluency. In order to determine the contribution of the y⁺ transporter for LA supplied to the cells, BAEC were incubated in an uptake buffer (HEPES, 25 mM; CaCl₂, 1.8 mM; KCl 5.4 mM, choline 140 mM; MgSO₄, 0.8 mM and glucose, 5mM) containing 20nM [³H]-LA for periods of 1, 2.5, 5, 15, 30 and 60 minutes. Uptake of LA was terminated by addition of ice cold buffer and cells were washed three times with 1 ml of buffer. After final washing, cells were lysed by adding 1 ml 0.5% sodium dodecyl sulfate in 0.1 N NaOH. Cellular lysates were added to 15 ml Ecoscint-A scintillation fluid, the amount of [³H]-LA was determined by scintillation spectroscopy (Beckman Instruments) and represented total cellular transport of LA. Cellular uptake of LA via B^{α+} system was determined by substituting sodium chloride for choline and incubating as described above. The difference in uptake determined for both transporters and that observed for y+ alone represented the amount of LA uptake contributed by sodium-dependent B^{α+} system. In addition, experiments were performed in which y+ and B^{α+} transporters were characterized for cellular uptake of [³H]-IA for 1, 2.5, 5, 15, 30 and 60 minutes in the presence of excess (10 mM) unlabeled LA. These results allowed us to determine to what extent non-specific cellular binding and diffusion contributed to apparent LA uptake and were subtracted from experimental values. It has been previously reported that passive, non-saturable diffusion of LA can occur but only when extracellular levels of LA are within the millimolar concentration.

*Cellular uptake of [*^{*3*}*H]-L-arginine in the presence of NOS agonists and NO donors*. BAEC were pre-incubated with NO agonists bradykinin (BK, µM), substance P (SP, 1 µM) and acetylcholine (Ach, 5 µM) or NO donors, S-nitroso-acetyl-penicillamine (SNAP, 200 µM; equivalent to 0.4 µM NO) and dipropylenetriamine NONOate (DPTA, 10-0.01 µM; equivalent to 20-0.02 µM NO) for 1, 2 or 4 hours. At the end of these treatment periods, cells were washed with uptake buffer and incubated for 1 hour in uptake buffer containing [³H]-LA. Other cells that were not pre-incubated, but rather acutely exposed to treatments for 2, 15, 30 or 60 minutes, were incubated with uptake buffer containing both triatiated LA and treatments for the duration of acute periods. Uptake of LA was terminated and total cellular transport of LA determined as described above.

*Cellular superoxide anion formation in the presence of NOS agonists*. The production of 0₂^{•-} by BAEC was determined by spectrophotometrically measuring the superoxide dismutase-inhibitable reduction of ferricytochrome C according to Pritchard *et al.,* 1995. BAEC were plated in 50 mm dishes containing 10.5 x 20 mm fibronectin-coated *Thermanox* coverslips. After reaching confluency, cells were washed 3 times (3 ml) with Dulbecco's phosphate buffered saline (DPBS) and 2 coverslips were placed in a disposable plastic cuvette facing each other. DPBS (1.8 ml) was gently placed in cuvette in addition to ferricytochrome C (final concentration, 50 µmol/L). The cuvette was inverted to mix the reagents and the absorbance was recorded for 60 minutes using a spectophotometer at 550 nm wavelength for cells alone (basal O₂^{•-} production) and cells stimulated with SP (1 µM) or A-23187 (1 µM). In order to determine whether supplemental extracellular LA or the LA analogue L-NAME could prevent or reduce the amount of superoxide anion formed, experiments were performed in which LA (5 x 10⁻⁴ M) and L-NANIE (5 x 10⁻⁴M) were added prior to treatments. Change in absorbance over time was determined and the amount of superoxide anion [ε=2100cm⁻¹ •(mol/L)⁻¹] generated was determined over time and reported as pmoles O₂^{•-}/min./10⁶ cells.

*Data analysis*. Data are expressed as means ±S.E.M. Comparisons of data between experimental groups with their appropriate controls were made using ANOVA or paired Student's t-test. Ap value of 0.05 or less was considered to represent a significant difference.

Fig. 2 is a schematic representation of the dynamics of LA supply to NOS. LA levels are maintained primarily through the activity of the carrier-mediated Na⁺-independent transporter, y⁺, while the Na⁺-dependent transporter, B^{α+}, and passive diffusion account for less than 15%. Concurrent transport of LA to NOS may control NO production. However, LA supply to NOS can be limiting due to compartmentalization within EC, arginase activity or utilization of LA by NOS. We believe that NO and superoxide anion reduce the activity of the y⁺ transporter and also reduce LA available for NOS. Collectively, summation of supply verses demand or availability of LA to NOS will determine whether NO or superoxide anion are formed.

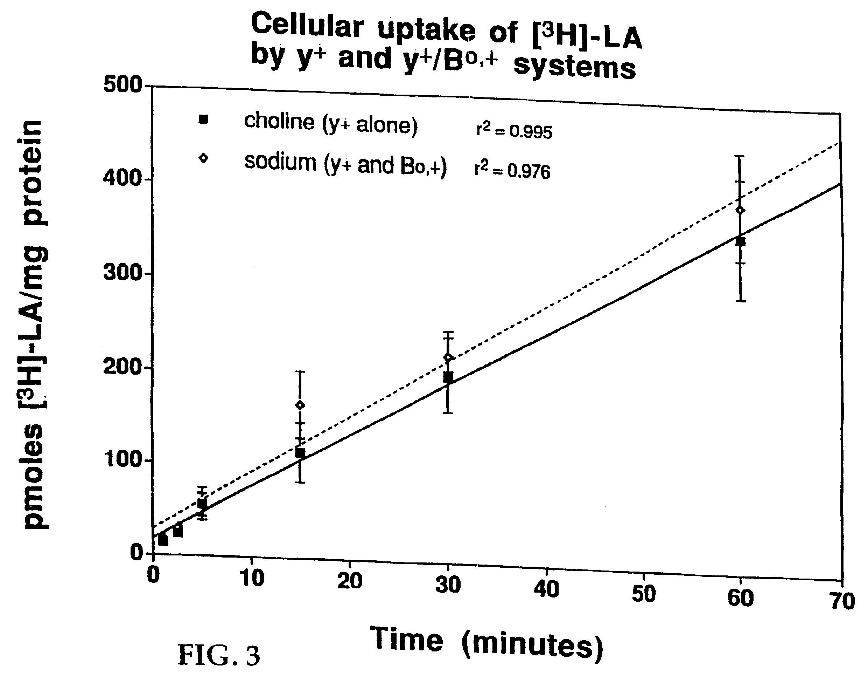

*Cellular Transport of LA into BAEC*. As can be seen in Fig. 3, initial data demonstrated that transport of cellular [³H]-LA, into BAEC occurs linearly with time for up to 1 hour. Fig. 3 indicates the effect of B^{α+} and y⁺ transporters on cellular uptake of [³H]-L-arginine. Bovine aortic endothelial cells were incubated with uptake buffer containing tritiated L-arginine and the amount of [³H]-L-arginine delivered to cells over time was determined as described in "Methods." Dashed line, uptake of [³H]-LA by both B^{α+} and y⁺ transporters; solid line, uptake of [³H]-LA by y⁺ transporter. Data are presented as mean ±S.E.M. We also found that the primary transporter of LA into these BAEC is the y+ transporter which was responsible for~85% of the [³H]-LA delivered to cells. The B^{α+} transporter system accounted for an average of 10% total transport. Passive diffusion as a percent of total cellular LA uptake was variable and decreased as the period of uptake was increased, accounting for 5 to 1.5% of total cellular LA transport during 15 to 60 minutes of uptake, respectively.

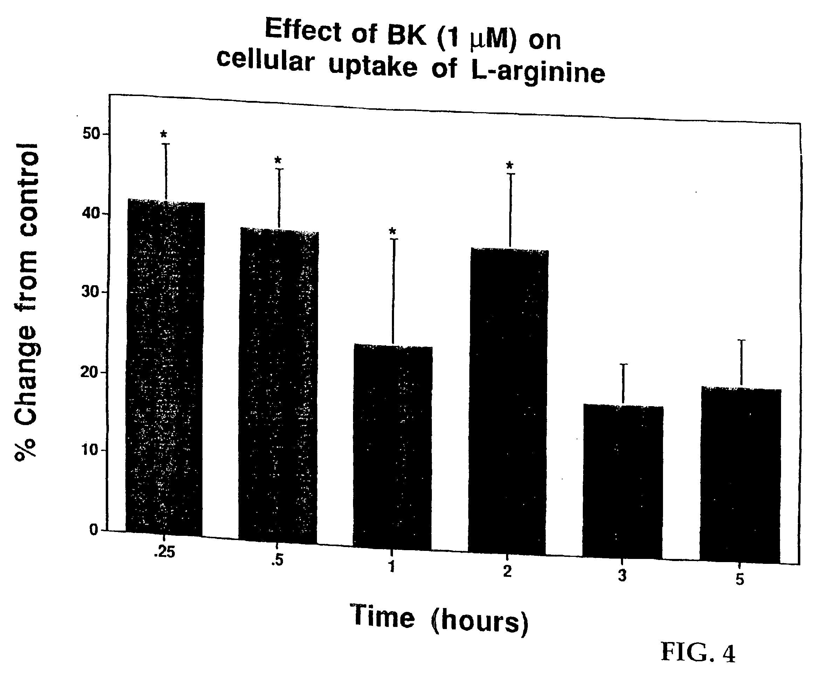

*Effect of NOS agonists on cellular uptake of LA*. As can be seen in Figs. 4, 5, and 6, LA transporter activity was augmented after acute exposure to select NOS agonists. Fig. 4 indicates the effect of bradykinin (BK, 1αM) on y⁺ transport of [³H]-LA in bovine aortic endothelial cells. Cells were exposed to BK and incubated with sodium-free uptake buffer containing tritiated LA and the amount of [³H]-LA delivered to cells was determined as described in "Methods." Data are presented as mean S.E.M; p<0.05 from control values. As can be seen in Fig. 4, bradykinin (BK) enhanced cellular transport of LA with maximum increase of 42% observed after 15 minute exposure and slightly less but still marked increases of 39 and 16% occurring after treatment for 30 and 60 minutes, respectively. Prolonged exposure of BAEC to BK enhanced cellular uptake of LA by 38% after 2 hour exposure. A similar magnitude of increase was also observed after 3 and 5 hour exposure, with increases in transport of 19 and 22%, respectively.

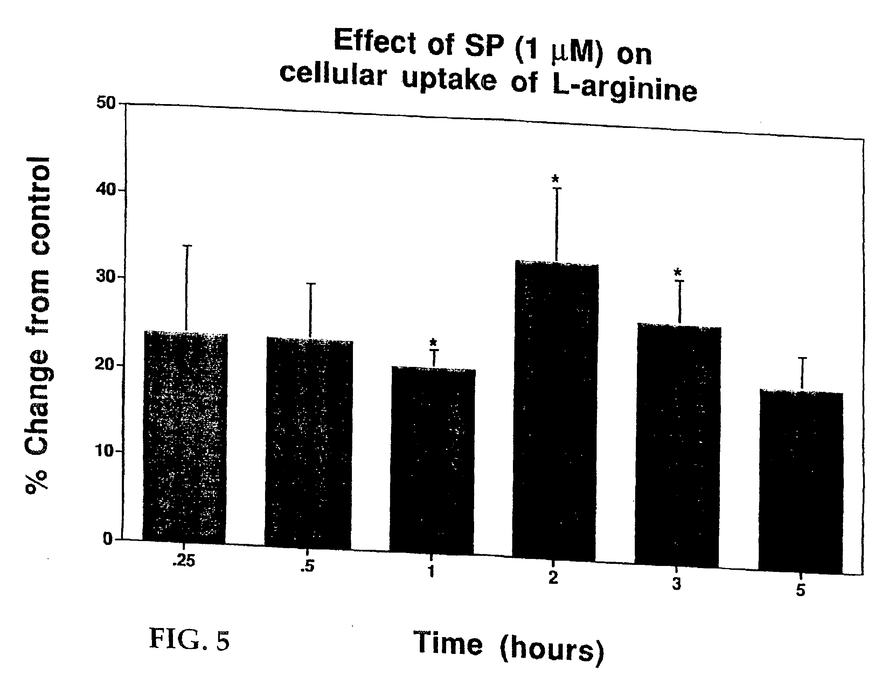

Fig. 5 indicates the effect substance P (SP, 1µM) on y⁺ transport of [³H]-LA in bovine aortic endothelial cells. Cells were exposed to SP and incubated with sodium-free uptake buffer containing tritiated LA and the amount of [³H]-LA delivered to cells was determined as described in "Methods." Data are presented as mean S.E.M; p<0.05 from control values. As can be seen in Fig. 5, substance P (SP) was also effective in augmenting cellular uptake of LA into cells. SP increased y⁺ transport of LA into cells by 24% after only 15 minutes exposure. This elevated LA uptake was maintained for exposures of 30 and 60 minutes with 24 and 21% increases, respectively. In addition, the effect of SP on cellular transport of [³H]-LA was enhanced after pre-treatment with SP for more prolonged durations. After 2 hour exposure of BAEC to SP, y⁺ transporter activity was enhanced as much as 34% from control values. This increase in transporter activity was also maintained after 3 and 5 hour exposure with cellular LA increases of 27 and 21%, respectively.

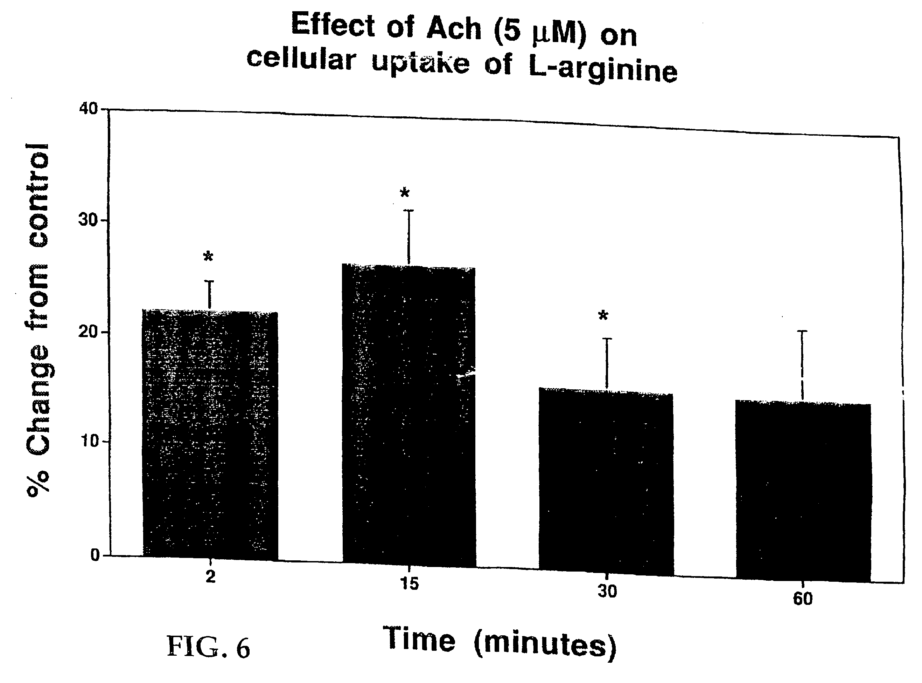

Effects of a third NOS agonist, acetylcholine (Ach) on the cellular uptake of [³H]-LA are shown in Fig. 6. Fig. 6 indicates the effect of acetylcholine (Ach, 5 µM) on y⁺ transport of [³H]-LA in bovine aortic endothelial cells. Cells were exposed to Ach and incubated with sodium-free uptake buffer containing tritiated LA and the amount of [³H]-LA delivered to cells was determined as described in "Methods." Data are presented as mean S.E.M; p<0.05 from control values. Incubation with Ach increased LA transport over all time periods. A 22% increase of [³H]-LA uptake was observed after 2 minute exposure to Ach. After 15 minute addition of Ach, LA uptake reached to a maximum increase of 27%. Treatment with Ach for 30 or 60 minutes resulted in 16 and 15.5% increases of LA uptake, respectively.

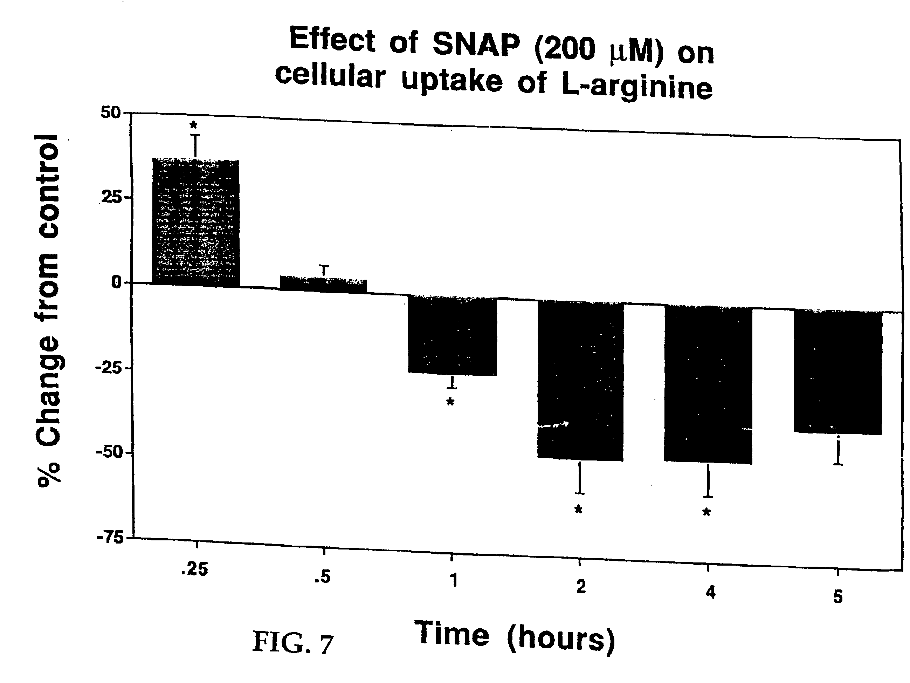

*Effect of NO donors on cellular uptake of LA*. Fig. 7 indicates the effect of s-nitroso-acetyl-penicillamin (SNAP, 200 µM; equivalent to 0.4 µM NO) on y⁺ transport of [³H]-LA in bovine aortic endothelial cells. Cells were exposed to SNAP and incubated with sodium-free uptake buffer containing tritiated LA and the amount of [³H]-LA delivered to cells was determined as described in "Methods." Data are presented as mean S.E.M; p<0.05 from control values. As can be seen in Fig. 7, treatment of endothelial cells with 200 µM SNAP (0.4 µM NO) markedly increased activity of the Y⁺ transporter by 37% occurring after ten minutes of exposure. This elevation was not seen after 30 minute exposure. By 1 hour, uptake of [³H]-LA was reduced by 22%. Inhibition was maintained with 46, 45, and 36% reductions observed after 2, 3 and 5 hour exposures to NO, respectively.

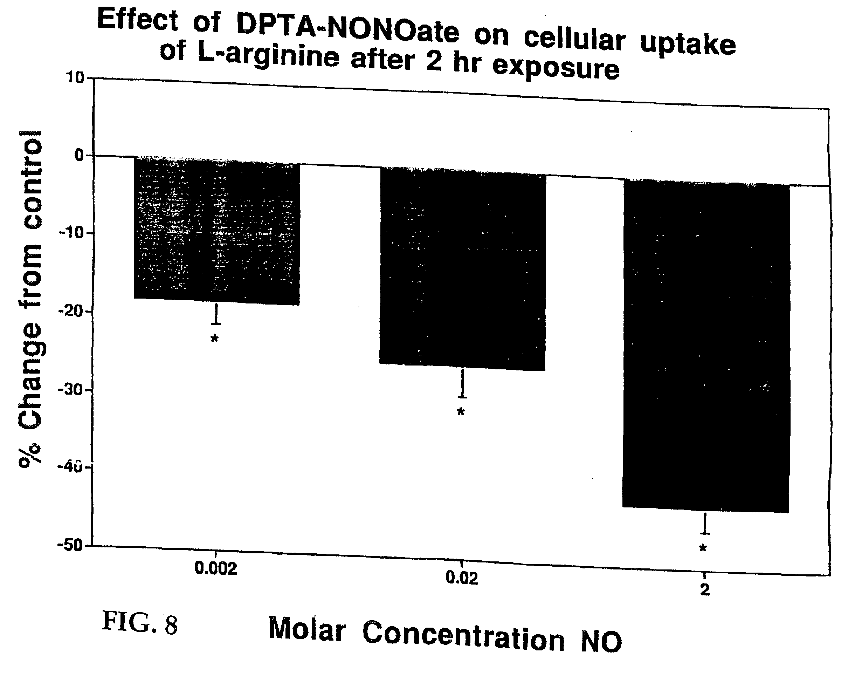

In order to confirm whether the reduction in cellular uptake of [³H]-LA was due to NO released from SNAP, experiments were performed using another NO donor, DPTA-NONOate. Unlike SNAP, which donates large amounts of NO over a short time period (t_{1/2}~10 minutes), the use of DPTA-NONOate allows for a slower (t_{1/2}~5 hours), more sustained release of NO that is constant over time. Fig. 8 indicates the effect of dipropylenetriamine NONOate (DPTA, 10-0.01 µM; equivalent to 20-0.02 µM NO) on y⁺ transport of [³H]-LA in bovine aortic endothelial cells. Cells were exposed to DPTA and incubated with sodium-free uptake buffer containing tritiated LA and the amount of [³H]-LA delivered to cells was determined as described in "Methods." Data are presented as mean S.E.M; p<0.05 from control values. Exposure to 1 µM DPTA-NONOate (2 µM NO) had no significant effect on y⁺ system at the earlier periods (15 and 30 min); however significant inhibitions of 22, 24 and 29% for LA transport were observed after 1, 2 and 4 hour exposures, respectively (data not shown). From Fig. 8, this repression appeared to be concentration dependent with maximum inhibition of 20, 24 and 44% occurring after 2 hour exposure with concentrations of 0.01, 1, and 10 µM (20, 2 and 0,02 µM NO), respectively.

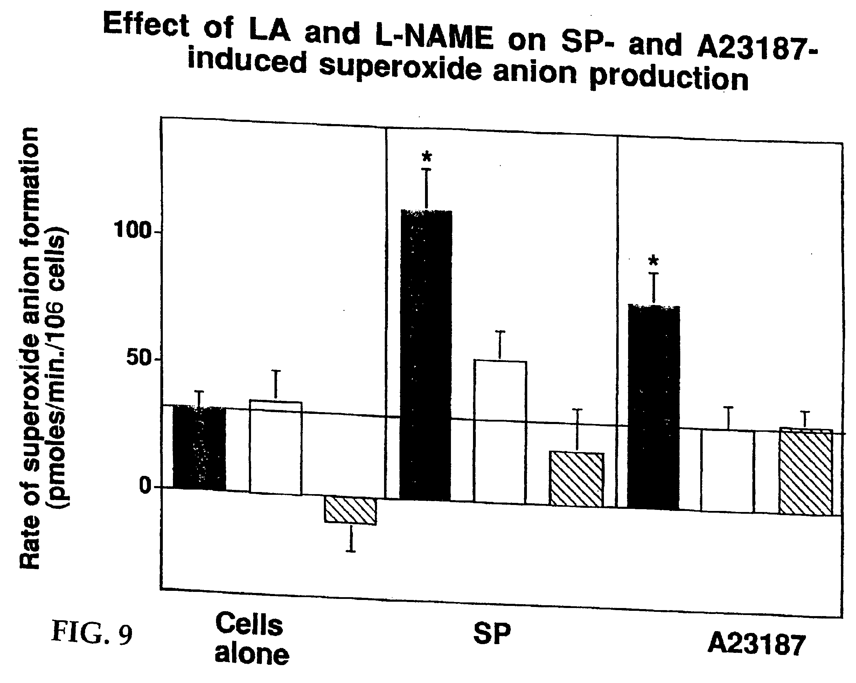

*Cellular superoxide anion formation - Effect of NOS agonists on cellular superoxide anion formation*. In order to determine the effects of extracellular LA or NOS antagonist L-NAME on BAEC superoxide anion formation, experiments were performed in which cellular production of superoxide anion was monitored alone (basal) and during treatment with SP (1 µM) or the calcuim ionophore A-23187 (1 µM, with or without concurrent presence of LA or L-NANIE supplementation. Fig. 9 indicates the effect of L-arginine (LA, 5 x 10-⁴M) and n-ω-nitro-L-arginine methyl ester (L-NAME, 5 x 10-⁴M) on substance P (SP, 1 µM) or calcium ionaphore, A-23187 (CI, 1 µM) induced superoxide anion (O₂^{•-}) formation in bovine aortic endothelial cells (BAEC). BAEC were treated with SP or A-23187 in the presence or absence of LA or L-NAME and O₂· production was determined over a 60 minute time period and compared to basal levels as described in "Methods." Data are presented as mean S.E.M; p<0.05 from control values. Fig. 9 demonstrates that O₂^{•-} is produced by BAEC and that supplementation with L-NAME, but not LA, prevented basal production of O₂^{•-} by 100%. Addition of SP or A-23187 significantly increased O₂· production above basal levels by 3.5 and 2.5 fold, respectively. Concurrent treatment with either LA (5 × 10⁻⁴M) or L-NAME (5 × 10⁻⁴M) effectively reduced O₂· induced by SP by 51 and 81%, respectively. Similar inhibitory effects of LA and L-NAME on O₂^{•-} production were observed when the calcium ionophore A-23187 was used to induce NOS activation, with 60 and 58% inhibition observed with LA and L-NAME, respectively.

The transport of LA to cells is critical for maintaining adequate LA levels such that optimal coupling of LA with endothelial NOS (eNOS) can occur. Therefore, factors affecting the y⁺ transporter system have the potential to limit the production of NO. Without ample LA, eNOS will solely utilize O₂ to form O₂^{•-} that may contribute to the pathogenesis of disease. As a consequence, controlling LA supply and other factors affecting superoxide production would be beneficial in normal as well as pathological circumstances.

The cellular LA transport system in BAEC is characterized here. The data presented herein confirms that the primary source of LA supply is through activity of the system y⁺ transporter and that delivery of LA into cells occurs linearly over two hours. In addition, we have verified that system B^{α+} transport activity and passive diffusion contribute minimally to the delivery of LA into BAEC under basal conditions. Our experimental results were similar to those observed using human umbilical EC and porcine aortic EC. These experiments were important to perform in order to determine which transport mechanism should be studied.

The data presented herein demonstrates BK causes an increase in cellular uptake of LA. These results are consistent with a study by Bogle *et al.* in which porcine aortic endothelial cells grown on microcarrier beads increased their cellular uptake of [³H]-LA in the presence of BK within 10 minutes. In addition to these findings, we were able to demonstrate that this enhancement of cellular uptake of LA was maintained from 15 minutes through 2 hours exposure to BK. More importantly, we were able to demonstrate an increase in y⁺ transporter activity for two other NOS agonists, SP and Ach. As stated earlier, a negative change in cellular membrane potential is thought to be the mechanism by which y⁺ system activity is maintained. Hyperpolarization associated with stimulation of y⁺ system is thought to occur by first increasing intracellular Ca⁺⁺. This increase in Ca⁺⁺-dependent potassium channels (K_{ca}) resulting in K+ efflux and hyperpolarization. Since BK, SP and Ach have also been shown to induce cellular hyperpolarization, these data suggest the increase in y⁺ transporter activity observed occurred by a similar mechanism.

Interestingly, our data for the NO donor, SNAP, depicts initial stimulation of the y⁺ transporter within 10 minutes followed by no change and then inhibition of cellular LA uptake with more prolonged exposures to NO, a "cross-over" effect. An initial increase of cellular uptake of LA is expected as NO is known to cause cellular hyperpolarization. However, longer exposures of 1 to 4 hours resulted in a marked reduction of LA transport. These data were confirmed by using a different NO donor, DPTA, to stimulate prolonged exposure of cells to NO. DPTA releases NO slowly over time and, therefore, was used to repeat the longer durations of NO exposure. Although one might expect to see a continued increase of y⁺ transporter activity with NO exposure similar to that observed using NOS agonists, there is evidence that oxidative properties of NO may be responsible for the reduction of cellular LA transport seen with longer exposure periods. It has been demonstrated that NO, through constant gas infusion and release from SNAP, decreases y⁺ system transporter activity. The negative effect of NO on y⁺ transport of LA into cells was determined to be associated with oxidation of sulfhydryl moieties in the transporter proteins since treatment with disulfide reducing agent dithiothreitol restored transporter activity. Furthermore, treatment of endothelial cells with sulfhydryl reactive chemicals N-ethylmaleimide (NEM) and acrolein reduced y⁺ transporter activity. Collectively, these data suggest that the effects of NO on cellular y⁺ LA transport activity are two-fold. The initial effect seen upon acute exposure is more likely due to the hyperpolarizing properties of NO while the latter inhibitory effects observed with more prolonged exposure to NO may be the result of a summation of cell hyperpolarizing and transport oxidizing properties of NO, the latter becoming more predominant.

The biphasic effect in transport function over time noted for SNAP was not observed in cells treated with prolonged exposure to NOS agonists. It would be expected that stimulation of NOS would also increase NO production and oxidation of the y⁺ transporter system resulting in inhibition of LA uptake similar to that observed with SNAP. One explanation for lack of biphasic action with NOS agonists could be that the amount of NO produced upon NOS activation is far less than the amount of NO released from SNAP. Therefore, levels of NOS derived NO never accumulate high enough for significant oxidation of the y⁺ transporter. Another possibility to explain the lack of inhibition of LA transport with NOS agonist is the fact that upon stimulation with NOS, LA is converted to the intermediate NG-hydroxyl-L-arginine (1-HOArg) prior to forming L-citrulline and NO. L-HOArg is known to be an antioxidant and an inhibitor of arginase. Therefore, the L-HOArg intermediate may provide protection from oxidation by newly formed NO. By preventing the metabolism of LA in the ornithine cycle, the net amount of LA available for eNOS may increase and lead to a reduction in O₂^{•-} formation. Both of these actions should protect the system y⁺ transporter from inactivation.

Hence the transport of LA into cells via y⁺ transport system may be unfavorably altered with elevated levels of NO. High concentrations of NO could occur during circumstances in which NOS is constantly stimulated. Pathophysiological conditions associated with increased NOS activity include hypoxia, hyperglycemia and hypertensive states mediated by elevations in angiotensin II (high renin essential and renovascular hypertension). The combination of increased NOS activity (LA demand) and decreased arginine uptake (LA supply) has the potential to create an LA deficiency ("demand-supply mismatch") which can result in the increased superoxide anion production seen in states such as ischemia-reperfusion injury. Increased O₂^{•-} production and NOS activity have also been shown to be associated with hyperglycemia.

The production of O₂^{•-} in BAEC alone and during treatment with NOS agonists is characterized herein. In addition, the effects of basal and NOS agonist induced O₂^{•-} production with concurrent addition of LA and L-NAME has been presented herein. The data demonstrate that BAEC produce O₂^{•-} which increases with time and supplementation with L-NAME reduces basal O₂^{•-} production. Since L-NAME is a selective NOS antagonist, this suggests that primary source of basal O₂^{•-} observed is from eNOS. Stimulation of BAEC with SP or A-23187 produced amounts of O₂^{•-} much greater than basal levels. Interestingly, a striking reduction of O₂^{•-} production was observed upon extracellular addition of either LA or L-NAME, following treatment with SP and A-23187. These data also suggest that excessive O₂^{•-} formation associated with agonist induced eNOS activation, but not basal production, can be ameliorated with LA supplementation.

Collectively, our findings strongly suggest that although intracellular LA levels far exceed the concentration of LA required by NOS for NO production, the amount of LA available for utilization by NOS can be insufficient especially in conditions of chronic eNOS stimulation. The explanation for this LA paradox may be provided by the work of McDonald and colleagues. Using porcine pulmonary artery endothelial cells with antibodies specific for caveolin, eNOS and the y⁺ transporter, McDonald *et al*. demonstrated that all of these proteins are co-localized within the plasma membrane caveolae. This suggests that eNOS associated with this complex is sequestered from overall intracellular LA and relies on the *de novo* transport of LA into the cell via the y⁺ transporter within the caveolae for NO production. If the transporter becomes damaged as seen with oxidation, LA supply could immediately become limiting and may be the basis for endothelial dysfunction. In addition, this eNOS/y⁺ transporter-caveolae complex may explain why endothelial dysfunction is quickly reversed with increasing extracellular LA. Once the transporter is turned off, LA concentration gradient increases and delivery of LA into cells is shifted towards passive diffusion. Therefore, extracellular supplementation of LA may be helpful in driving passive diffusion of LA when the integrity of carrier-mediated transporters cannot be maintained.

In conclusion, we believe that concurrent LA supply to NOS via system y⁺, independent of overall intracellular LA, is critical in establishing and maintaining vascular function. Factors including NOS agonists and NO itself appear to control y⁺ activity and the summation of these factors is critical in determining NO and superoxide anion formation, both of which contribute to vascular dysfunction and disease.

Therefore, it would appear that combining L-arginine with other antioxidants, including nutraceuticals, may provide the most beneficial results when treating diseases associated with superoxide anion production.

Two nutraceuticals of particular interest are gingko biloba and hawthorne extract. Gingko biloba has been reported in scientific journals to enhance blood circulation and to increase the supply of oxygen to the heart, brain and other body parts. This ability to enhance blood circulation and increase oxygen supplies is believed to make it useful for improving memory and relieving muscle pain. It also acts as an antioxidant, has anti-aging effects, reduces blood pressure, inhibits blood clotting, and is helpful for tinnitus, vertigo, hearing loss, impotence, and Raynod's Disease. Gingko biloba has even been shown to slow the early progression of Alzheimer's Disease in some individuals.

Hawthorne extract is a nutraceutical that has been shown to dilate the coronary blood vessels, lower cholesterol levels, and restore heart muscle. It is also known to increase intracellular vitamin C levels. Hawthorne extract is therefore thought to be useful for anemia, cardiovascular and circulatory disorders, hypercholesterolemia and for enhancing immunity status.

A third aspect of the present invention is the treatment, prevention, or amelioration of homocysteinemia with L-arginine alone. Hyperhomocysteinemia is associated with vascular EC dysfunction and increased risk for atherosclerosis and atherothrombosis. Free radical formation during homocyst(e)ine auto-oxidation has been implicated in its vascular toxicity. Long-term EC exposure to homocyst(e)ine limits nitric oxide synthase (NOS) activity, increasing EC vulnerability to oxidative injury (Stamler *et al.,* 1993). To see whether these effects could be due to alterations in cellular availability of the NOS substrate LA, we tested the effects on LA transport of D,L-homocysteine (DLH) and an auto-oxidation product, L-homocysteine thiolactone (LHT). Bovine aortic EC were exposed to DLH and LHT (1.1-5 mM) for 6 and 24 hours and then incubated with 50 mM [³H]-LA for 1 min. Cells were washed with ice cold buffer, lysed in 0.5% SDS in 0.1 N NaOH and radioactivity was determined. DLH increased LA uptake in a concentration dependent manner at 6 hr. The maximum increase of 53% was observed at 5 mM of DLH. However, after 24 hr. exposure LA uptake was decreased (25-40%). LHT produced a dose-related decrease in LA transport after 6 and 24 hrs exposure.

In conclusion, DLH produced biphasic effects on LA transport over time. Initial increases in LA transport (6 hr) may be due to enhanced eNOS activity, since short-term DLH treatment has been found to induce a dose-dependent increase in NO production to NOS agonists (Upchurch *et al.,* 1997). In contrast, longer DLH exposure and exposure to the oxidation product LHT for 6 and 24 hr suppress LA transport function. This reduction in LA availability can exacerbate EC dysfunction by shifting NOS activity from NO production to superoxide formation. This aspect of the invention supports a method of treating hyperhomocysteinemia with L-arginine alone or in combination with other therapeutic agents.

Although the preferred embodiments have been described in detail, it should be understood that various changes, substitutions, and alterations can be made in the present invention as defined by the claims appended hereto. For example, the aforementioned therapeutic mixture of L-arginine and DOX may alternatively be infused into a catheter used to diagnose peripheral vascular disease in patients suffering from claudication. The present invention is to be defined only by the claims attached hereto.

While the foregoing has been set forth in considerable detail, the sequences are presented for elucidation, and not limitation. Modifications and improvements, including equivalents, of the technology disclosed above which are within the purview and abilities of those in the art are included within the scope of the claims appended hereto. It will be readily apparent to those skilled in the art that numerous modifications, alterations and changes can be made with respect to the specifics of the above description without departing from the inventive concept described herein.

## Claims

1. A formulation comprised of non-pharmaceutical or nutraceutical agonist of NOS and a biological equivalent of L-arginine, said agonist being selected from the group consisting of hawthorne extract, gingko biloba, allicin (garlic), melatonin, folic acid, elderberry extract, grape pip riboflavin, phytoestrogens, soy isoflavones, resveraterol and quercetin.

2. A mixture comprised of L-arginine and a NOS agonist, said NOS agonist being a selective alpha-1 blocker.

3. A method of treating a diseased state comprised of administering a mixture of L-arginine and an agonist of NOS.

4. The method of claim 3 further comprising the administration of a selective alpha-1 blocker.

5. The method of claim 4, wherein said selective alpha-1 blocker is prazosin.

6. The method of claim 5, wherein said selective alpha-1 blocker is terazosin.

7. A therapeutic mixture of doxazosin and a substrate of NOS.

8. The therapeutic mixture of claim 7, wherein said substrate of NOS is a biological equivalent of L-arginine.

9. The therapeutic mixture of claim 7, wherein said substrate of NOS is L-arginine.

10. A method of treating a disease condition in a subject by vasodilation or vasorelaxation comprising:
- selecting a subject;
- administering a mixture of L-arginine and doxazosin;
- obtaining periodic indicators of vasorelaxations for the subject; and
- continuing administration of the mixture until a desirable state of vasorelaxation is obtained.

11. The method of claim 10, wherein the mixture is administered intravenously, buccal, intracoronary, intraarterially, intramuscularly, topically, intranasally, rectally, sublingually, orally, subcutaneously, by patch or inhalation.

12. The method of claim 10, wherein said disease is hypertension, hypercholesterolemia, hypertensive heart disease, coronary heart disease, cardiovascular disease, cerebrovascular disease, and renovascular disease.

13. The method of claim 12, wherein said coronary heart disease is restenosis post angioplasty.

14. The method of claim 10, wherein L-arginine and doxazosin are mixed in vivo.

15. The method of claim 10, wherein L-arginine and doxazosin are administered at a therapeutic concentration.

16. The method of claim 15, wherein the therapeutic concentration of L-arginine is from 7.5% to about 30% w/v (g/ml).

17. A method of stimulating nitric oxide synthase to produce nitric oxide, said method comprising:
- administering L-arginine and an agonist of nitric oxide synthase to a subject have a nitric oxide synthase receptor site, said agonist being doxazosin; and
- stimulating said nitric oxide synthase to a desirable level with said agonist of nitric oxide synthase.

18. The method of claim 17, wherein said L-arginine is in excess of doxazosin.

19. The method of claim 17, wherein a therapeutically effective amount of L-arginine is combined with a therapeutically effective amount of doxazosin prior to administration to the patient.
